(19) Europäisches Patentamt
European Patent Office
Office européen des brevets

(11) Veröffentlichungsnummer: **0 657 418 A1**

(12) # EUROPÄISCHE PATENTANMELDUNG

(21) Anmeldenummer: **94118847.6**

(22) Anmeldetag: **30.11.94**

(51) Int. Cl.6: **C07C 237/42**, C07D 309/08, A01N 37/22, A01N 43/16

(30) Priorität: **09.12.93 DE 4342026**

(43) Veröffentlichungstag der Anmeldung: **14.06.95 Patentblatt 95/24**

(84) Benannte Vertragsstaaten: **AT BE CH DE DK ES FR GB GR IE IT LI NL PT SE**

(71) Anmelder: **BASF AKTIENGESELLSCHAFT**

**D-67056 Ludwigshafen (DE)**

(72) Erfinder: **Eicken, Karl, Dr.**
**Am Hüttenwingert 12**
**D-67157 Wachenheim (DE)**
Erfinder: **Wagner, Oliver, Dr.**
**Siemensstrasse 1**
**D-66450 Bexbach (DE)**
Erfinder: **Rang, Harald, Dr.**
**Ziegeleistrasse 76**
**D-67122 Altrip (DE)**
Erfinder: **Ammermann, Eberhard, Dr.**
**Von-Gagern-Strasse 2**
**D-64646 Heppenheim (DE)**
Erfinder: **Lorenz, Gisela, Dr.**
**Erlenweg 13**
**D-67434 Neustadt (DE)**

(54) **Acylamino-benzamide und sie enthaltende fungizide Mittel.**

(57) Acylamino-benzamide I

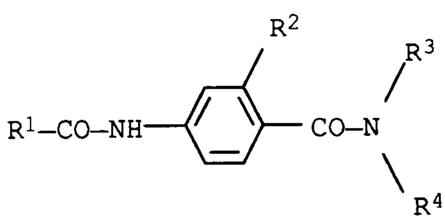

I

($R^1$ = Bicyclo[4.1.0]hept-1-yl, Bicyclo[3.1.0]hex-1-yl,
2-($C_1$-$C_3$-Alkyl)-bicyclo[2.2.2]oct-2-yl,
2-($C_1$-$C_3$-Alkyl)-bicyclo[2.2.2]oct-5-en-2-yl,
2-($C_1$-$C_3$-Alkyl)-bicyclo[2.2.1]hept-2-yl,
2-($C_1$-$C_3$-Alkyl)-bicyclo[2.2.1]hept-5-en-2-yl,
6-($C_1$-$C_3$-Alkyl)-5,6-dihydro-4H-pyran-6-yl oder
2-($C_1$-$C_3$-Alkyl)-2.3.5.6-tetrahydro-4H-pyran-2-yl;
$R^2$ = Halogen, CN, $NO_2$, $C_1$-$C_4$-Alkyl, $C_2$-$C_4$-Alkenyl, $C_2$-$C_4$-Alkinyl, $C_1$-$C_4$-Halogenalkyl, $C_1$-$C_4$-Alkoxy, $C_1$-$C_4$-Alkoxycarbonyl, $C_1$-$C_4$-Alkylcarbonyl, CHO oder Carboxy;
$R^3$,$R^4$ = CN, $C_1$-$C_4$-Alkyl, $C_1$-$C_4$-Halogenalkyl, $C_3$-$C_6$-Alkenyl, $C_3$-$C_6$-Halogenalkenyl, $C_3$-$C_6$-Alkinyl, $C_3$-$C_6$-Halogenalkinyl, $C_1$-$C_4$-Alkoxy, $C_1$-$C_4$-Alkoxy-$C_1$-$C_4$-alkyl, $C_1$-$C_4$-Alkylthio-$C_1$-$C_4$-alkyl, geg. subst. $C_3$-$C_6$-Cycloalkyl oder $C_3$-$C_6$-Cycloalkyl-$C_1$-$C_4$-alkyl, oder
$R^3$ + $R^4$ zusammen mit dem gemeinsamen N-Atom = geg. subst. Azetidin-, Pyrrolidin-, Piperidin- oder Morpholin-Ring).

EP 0 657 418 A1

Die vorliegende Erfindung betrifft neue Acylamino-benzamide der Formel I

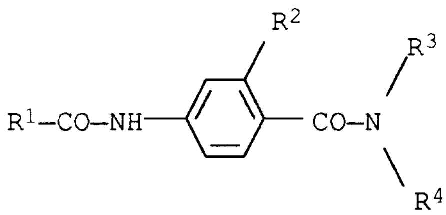

I

in der die Substituenten folgende Bedeutung haben:

- $R^1$ Bicyclo[4.1.0]hept-1-yl, Bicyclo[3.1.0]hex-1-yl, 2-($C_1$-$C_3$-Alkyl)-bicyclo [2.2.2]oct-2-yl, 2-($C_1$-$C_3$-Alkyl)-bicyclo[2.2.2]oct-5-en-2-yl, 2-($C_1$-$C_3$-Alkyl)-bicyclo[2.2.1]hept-2-yl, 2-($C_1$-$C_3$-Alkyl)-bicyclo[2.2.1]hept-5-en-2-yl, 6-($C_1$-$C_3$-Alkyl)-5,6-dihydro-4H-pyran-6-yl oder 2-($C_1$-$C_3$-Alkyl)-2.3.5.6-tetrahydro-4H-pyran-2-yl;
- $R^2$ Halogen, Cyano, Nitro, $C_1$-$C_4$-Alkyl, $C_2$-$C_4$-Alkenyl, $C_2$-$C_4$-Alkinyl, $C_1$-$C_4$-Halogenalkyl, $C_1$-$C_4$-Alkoxy, $C_1$-$C_4$-Alkoxycarbonyl, $C_1$-$C_4$-Alkylcarbonyl, Formyl oder Carboxy;
- $R^3$,$R^4$ unabhängig voneinander Cyano, $C_1$-$C_4$-Alkyl, $C_1$-$C_4$-Halogenalkyl, $C_3$-$C_6$-Alkenyl, $C_3$-$C_6$-Halogenalkenyl, $C_3$-$C_6$-Alkinyl, $C_3$-$C_6$-Halogenalkinyl, $C_1$-$C_4$-Alkoxy, $C_1$-$C_4$-Alkoxy-$C_1$-$C_4$-alkyl, $C_1$-$C_4$-Alkylthio-$C_1$-$C_4$-alkyl, $C_3$-$C_6$-Cycloalkyl oder $C_3$-$C_6$-Cyclo-alkyl-$C_1$-$C_4$-alkyl, wobei die Cycloalkylringe der letzten beiden Substituenten unsubstituiert sein oder ihrerseits ein bis drei $C_1$-$C_4$-Alkyl-reste tragen können, oder
- $R^3$ und $R^4$ zusammen mit dem Stickstoffatom, an das sie gebunden sind, einen Azetidin-, Pyrrolidin-, Piperidin- oder Morpholin-Ring, wobei die Ringe unsubstituiert sein oder ein bis zwei $C_1$-$C_4$-Alkylreste tragen können.

Außerdem betrifft die Erfindung Verfahren zur Herstellung dieser Verbindungen, ihre Verwendung als Fungizide, Fungizide Mittel, welche diese Verbindungen als wirksame Substanzen enthalten, sowie Verfahren zur Bekämpfung von Schadpilzen.

Aus der EP-A 381 330 sind bereits fungizid wirksame Acylaminobenzamide vom Typ der Verbindungen I bekannt.

Die fungiziden Wirkungen dieser Verbindungen insbesondere gegen Phycomyceten können jedoch, besonders bei kleinen Aufwandmengen und -konzentrationen, nur bedingt befriedigen.

Der vorliegenden Erfindung lag daher die Aufgabe zugrunde, neue fungizid wirksame Verbindungen mit verbessertem Wirkungsspektrum, insbesondere gegen Phycomyceten, zu finden.

Demgemäß wurden die eingangs definierten Acylamino-benzamide I gefunden. Weiterhin wurden Verfahren zur Herstellung dieser Verbindungen, sie enthaltende Mittel und Verfahren zur Bekämpfung von Schadpilzen mit diesen Mitteln gefunden.

Die vorstehend für die Substituenten $R^2$, $R^3$ und $R^4$ genannten Bedeutungen stellen Sammelbegriffe für individuelle Aufzählungen der einzelnen Gruppenmitglieder dar. Sämtliche Kohlenstoffketten, also alle Alkyl-, Alkylcarbonyl-, Alkenyl-, Alkinyl-, Halogenalkyl-, Halogenalkenyl-, Halogenalkinyl-, Alkoxy-, Alkoxycarbonyl- und Alkylthio-Teile können geradkettig oder verzweigt sein. Halogenierte Substituenten tragen vorzugsweise ein bis fünf gleiche oder verschiedene Halogenatome.

Im einzelnen bedeuten beispielsweise:

- Halogen: Fluor, Chlor, Brom und Jod, vorzugsweise Fluor und Chlor;
- $C_1$-$C_4$-Alkyl; Methyl, Ethyl, n-Propyl, 1-Methylethyl, n-Butyl, 1-Methylpropyl, 2-Methylpropyl und 1,1-Dimethylethyl, vorzugsweise n-Propyl, 1-Methylethyl, n-Butyl, 1-Methylpropyl, 2-Methylpropyl, 1,1-Dimethylethyl;
- $C_2$-$C_4$-Alkenyl: Ethenyl, Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl und 2-Methyl-prop-2-en-1-yl, vorzugsweise Vinyl, Allyl, 2-Methylallyl;
- $C_3$-$C_6$-Alkenyl: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl, vorzugsweise Ethenyl und Prop-2-en-1-yl;

- $C_2$-$C_4$-Alkinyl: Ethinyl, Prop-1-in-1-yl, Prop-2-in-3-yl, n-But-1-in-1-yl, n-But-1-in-4-yl und n-But-2-in-1-yl, vorzugsweise Ethinyl-, 2-Propinyl und 3-Butinyl;
- $C_3$-$C_6$-Alkinyl: Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise Ethinyl und Prop-2-in-1-yl;
- $C_1$-$C_4$-Halogenalkyl: $C_1$-$C_4$-Alkyl wie vorstehend genannt, das partiell oder vollständig durch Fluor, Chlor und/oder Brom substituiert ist, also z.B, Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 3-Chlorpropyl, vorzugsweise Trifluormethyl;
- $C_1$-$C_4$-Alkoxy: Methoxy, Ethoxy, n-Propoxy, 1-Methylethoxy, n-Butoxy, 1-Methyl-propoxy, 2-Methyl-propoxy, und 1,1-Dimethylethoxy, vorzugsweise Methoxy und Ethoxy;
- $C_1$-$C_4$-Alkoxycarbonyl: Methoxycarbonyl, Ethoxycarbonyl, n-Propoxycarbonyl, 1-Methylethoxycarbonyl, n-Butoxycarbonyl, 1-Methyl propoxycarbonyl, 2-Methylpropoxycarbonyl, und 1,1-Dimethyl-ethoxycarbonyl, vorzugsweise Methoxycarbonyl und Ethoxycarbonyl;
- $C_1$-$C_4$-Alkylcarbonyl: Methylcarbonyl, Ethylcarbonyl, n-Propylcarbonyl, 1-Methyl-ethylcarbonyl, n-Butylcarbonyl, 1-Methylpropyl-carbonyl, 2-Methylpropylcarbonyl und 1,1-Dimethylethylcarbonyl, vorzugsweise Methylcarbonyl und Ethylcarbonyl;
- $C_3$-$C_6$-Halogenalkenyl: $C_3$-$C_6$-Alkenyl wie vorstehend genannt, das partiell oder vollständig durch Fluor, Chlor und/oder Brom substituiert ist, also z.B. 2-Chlorallyl und 3-Chlor-prop-2-en-1-yl;
- $C_3$-$C_6$-Halogenalkinyl: $C_3$-$C_6$-Alkinyl wie vorstehend genannt, wobei jeweils ein bis drei Wasserstoffatome durch Fluor, Chlor und/oder Brom, insbesondere Fluor und Chlor, ersetzt sind, also z.B. 3-Chlor-2-propinyl;
- $C_3$-$C_6$-Cycloalkyl: Cyclopropyl, Cyclobutyl, Cyclopentyl und Cyclohexyl, vorzugsweise Cyclopropyl, Cylopentyl und Cyclohexyl, wobei die Cycloalkylringe ein bis drei $C_1$-$C_4$-Alkylgruppen wie vorstehend genannt tragen können;
- $C_3$-$C_6$-Cycloalkyl-$C_1$-$C_4$-alkyl: z.B. Cyclopropylmethyl, Cyclobutylmethyl, Cyclopentylmethyl, Cyclohexylmethyl, 2-(Cyclopropyl)ethyl, 2-(Cyclobutyl)ethyl, 2-(Cyclopentyl)ethyl, 2-(Cyclohexyl)ethyl, 3-(Cyclopropyl)propyl, 3-(Cyclobutyl)propyl, 3-(Cyclopentyl)propyl, 3-(Cyclohexyl)propyl, 4-(Cyclopropyl)-butyl, 4-(Cyclobutyl)butyl, 4-(Cyclopentyl)butyl und 4-(Cyclohexyl) butyl, vorzugsweise Cyclopropylmethyl und Cyclobutylmethyl, wobei die Cycloalkylringe ein bis drei $C_1$-$C_4$-Alkylgruppen wie vorstehend genannt, tragen können;
- $C_1$-$C_4$-Alkoxy-$C_1$-$C_4$-alkyl: Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, 2-Methoxyethyl, 2-Ethoxyethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)-ethyl, 2-(Methoxy)propyl, 3-(Methoxy)propyl, 2-(Ethoxy)propyl, 3-(Ethoxy)propyl, 3-Propoxypropyl, 3-Butoxypropyl, 4-(Methoxy)butyl, 4-(Ethoxy)butyl, 4-(n-Propoxy)butyl und 4-(n-Butoxy)butyl, vorzugsweise 2-Methoxyethyl;
- $C_1$-$C_4$-Alkylthio-$C_1$-$C_4$-alkyl: Methylthiomethyl, Ethylthiomethyl, n-Propylthiomethyl, (1-Methylethylthio)methyl, n-Butylthio-methyl, (1-Methyl-propylthio)methyl, (2-Methylpropylthio)methyl, (1,1-Dimethylethylthio, 2-Methylthioethyl, 2-Ethylthioethyl, 2- (n-Propylthio)ethyl, 2-(1-Methylethylthio)ethyl, 2-(n-Butylthio)ethyl, 2-(1-Methyl-propylthio)ethyl, 2-(2-Methylpropylthio)ethyl, 2-(1,1-Dimethylethylthio)ethyl, 2-(Methylthio)-propyl, 3-(Methylthio)propyl, 2-(Ethylthio)propyl, 3-(Ethylthio)propyl, 3-(Propylthio)-

propyl, 3-(Butylthio)propyl, 4-(Methylthio)butyl, 4-(Ethylthio)butyl, 4-(n-Propylthio)butyl und 4-(n-Butylthio)butyl, vorzugsweise 2-(Methylthio)ethyl;

$R^3$ und $R^4$ können zusammen mit dem Stickstoffatom, an das sie gebunden sind, einen Azetidin-, Pyrrolidin-, Piperidin- oder Morpholin-Ring bilden, wobei diese Ringe unsubstituiert sein oder ein bis zwei $C_1$-$C_4$-Alkylreste tragen können; bevorzugt sind Azetidin und Pyrrolidin.

Im Hinblick auf die Verwendung der Acylamino-benzamide I als fungizide Verbindungen stehen $R^2$ vorzugsweise für Chlor, Brom, Jod, Cyano, Nitro, Methyl, Vinyl, Ethinyl, Trifluormethyl, Methoxy, Methoxycarbonyl oder Acetyl und $R^3$ und $R^4$ jeweils beide für Methyl, Ethyl, Isopropyl, Allyl, Propargyl, Methoxymethyl, Cyclopropyl oder Cyclopropylmethyl.

In der folgenden Tabelle 1 sind ganz besonders bevorzugte Acylamino-benzamide I aufgeführt:

Tabelle 1

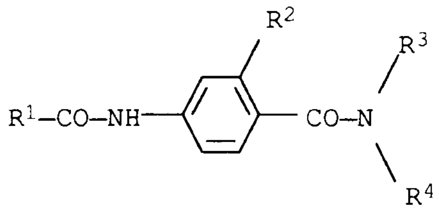

I

| Nr. | $R^1$ | $R^2$ | $R^3$ | $R^4$ |
|---|---|---|---|---|
| 01 | Bicyclo[4.1.0]hept-1-yl | Cl | $CH_3$ | $CH_3$ |
| 02 | Bicyclo[3.1.0]hex-1-yl | Cl | $CH_3$ | $CH_3$ |
| 03 | 2-Methyl-bicyclo[2.2.2]oct-2-yl | Cl | $CH_3$ | $CH_3$ |
| 04 | 2-Methyl-bicyclo[2.2.2]oct-5-en-2-yl | Cl | $CH_3$ | $CH_3$ |
| 05 | 2-Methyl-bicyclo[2.2.1]hept-2-yl | Cl | $CH_3$ | $CH_3$ |
| 06 | 2-Methyl-bicyclo[2.2.1]hept-5-en-2-yl | Cl | $CH_3$ | $CH_3$ |
| 07 | 6-Methyl-5,6-dihydro-4H-pyran-6-yl | Cl | $CH_3$ | $CH_3$ |
| 08 | 2-Methyl-2.3.5.6-tetrahydro-4H-pyran-2-yl | Cl | $CH_3$ | $CH_3$ |
| 09 | Bicyclo[4.1.0]hept-1-yl | Br | $CH_3$ | $CH_3$ |
| 10 | 2-Methyl-bicyclo[2.2.2]oct-2-yl | Br | $CH_3$ | $C_2H_5$ |
| 11 | 2-Methyl-bicyclo[2.2.1]hept-2-yl | Br | $CH_3$ | $CH_3$ |
| 12 | 2-Methyl-2.3.5.6-tetrahydro-4H-pyran-2-yl | Br | $CH_3$ | $CH_3$ |
| 13 | Bicyclo[4.1.0]hept-1-yl | $CH_3$ | $CH_3$ | $CH_3$ |
| 14 | 2-Methyl-bicyclo[2.2.2]oct-2-yl | $CH_3$ | $CH_3$ | $CH(CH_3)_2$ |
| 15 | 2-Methyl-bicyclo[2.2.1]hept-2-yl | $CH_3$ | $CH_3$ | $CH_3$ |
| 16 | 2-Methyl-2.3.5.6-tetrahydro-4H-pyran-2-yl | $CH_3$ | $CH_3$ | $CH_3$ |
| 17 | Bicyclo[4.1.0]hept-1-yl | Cl | $CH_3$ | $C_2H_5$ |
| 18 | 2-Methyl-bicyclo[2.2.2]oct-2-yl | Cl | $CH_3$ | $CH_2C\equiv CH$ |
| 19 | 2-Methyl-bicyclo[2.2.1]hept-2-yl | Cl | $CH_3$ | $CH_2-CH=CH_2$ |
| 20 | 2-Methyl-2.3.5.6-tetrahydro-4H-pyran-2-yl | Cl | $CH_3$ | $CH_2OCH_3$ |
| 21 | Bicyclo[4.1.0]hept-1-yl | J | $CH_3$ | $CH_3$ |
| 22 | 2-Methyl-bicyclo[2.2.2]oct-2-yl | $CH=CH_2$ | $CH_3$ | $CH_3$ |

| Nr. | $R^1$ | $R^2$ | $R^3$ | $R^4$ |
|---|---|---|---|---|
| 23 | 2-Methyl-bicyclo[2.2.1]hept-2-yl | C≡CH | $CH_3$ | $CH_3$ |
| 24 | 2-Methyl-2.3.5.6-tetrahydro-4H-pyran-2-yl | CN | $CH_3$ | $CH_3$ |

Die Acylamino-benzamide I sind auf verschiedene Weise erhältlich, und zwar vorzugsweise nach den folgenden Methoden:

a) Umsetzung von Carbonsäurehalogeniden $R^1$-CO-Cl mit Anilinen II auf an sich bekannte Weise in Gegenwart einer Base (vgl. z.B. J. March, Advanced Organic Chemistry, 2nd Ed., 382 f, McGraw-Hill, 1977):

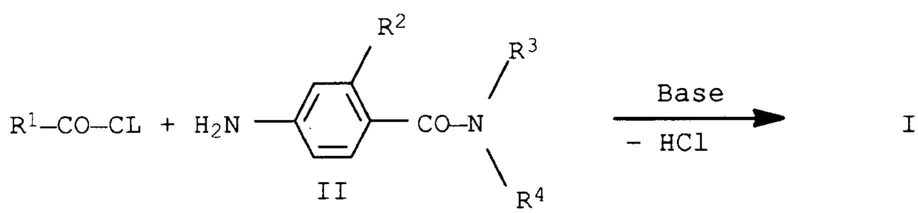


Die Reaktionsführung erfolgt normalerweise in einem inerten Lösungs- oder Verdünnungsmittel, beispielsweise in aromatischen Kohlenwasserstoffen wie Toluol und Xylol, in halogenierten Kohlenwasserstoffen wie Dichlormethan und Chloroform, in Ethern wie Diethylether und Tetrahydrofuran, in Estern wie Essigsäureethylester oder in Ketonen wie Aceton.

Als Basen eignen sich besonders organische Basen, z.B. tert.-Amine wie Triethylamin und Pyridin.

Üblicherweise verwendet man mindestens 1 Mol-Äquivalent an Base, bevorzugt 1,0 bis 1,1 Mol-Äquivalente, bezogen auf die Menge an II.

Im allgemeinen liegt die Reaktonstemperatur bei (- 20) bis 100 °C, vorzugswesie bei 0 bis 50 °C.

Zweckmäßigerweise setzt man die Ausgangsverbindungen $R^1$-CO-Cl und II im stöchiometrischen Verhältnis ein, jedoch kann in manchen Fällen auch ein Überschuß der einen oder anderen Komponente, etwa bis zu 10 mol-%, empfehlenswert sein.

Die Säurechloride $R^1$-CO-Cl lassen sich ihrerseits aus bekannten Säuren oder Estern (vgl. J. Org. Chem. 35, 2666 (1970), J. Org. Chem. 50, 2128 (1985), J. Amer. Chem Soc. 73, 5270 (1951), Houben-Weyl Methoden der Organ. Chemie Bd. 5/1c, S. 991ff. (1970)) herstellen.

Die Aniline der Formel II erhält man vorteilhaft auf an sich bekannte Weise durch Reduktion von Nitroverbindungen der Formel III. Als Reduktionsmittel hierfür eignen sich beispielsweise Eisen in Eisessig oder in Wasser mit Zusatz von Ethanol oder Wasserstoff unter Verwendung eines Edelmetallkatalysators wie Nickel, Palladium und Platin.

Die Nitroverbindungen der Formel III sind ihrerseits z.B. aus Nitrobenzoesäuren der Formel V herstellbar, die zuerst mit üblichen Chlorierungsmitteln (z.B. Phosgen, Thionylchlorid) in die entsprechenden Säurechloride IV überführt werden wonach diese mit einem Amin $HNR^3R^4$ in einem geeigneten Lösungsmittel in Gegenwart einer Base umgesetzt werden:

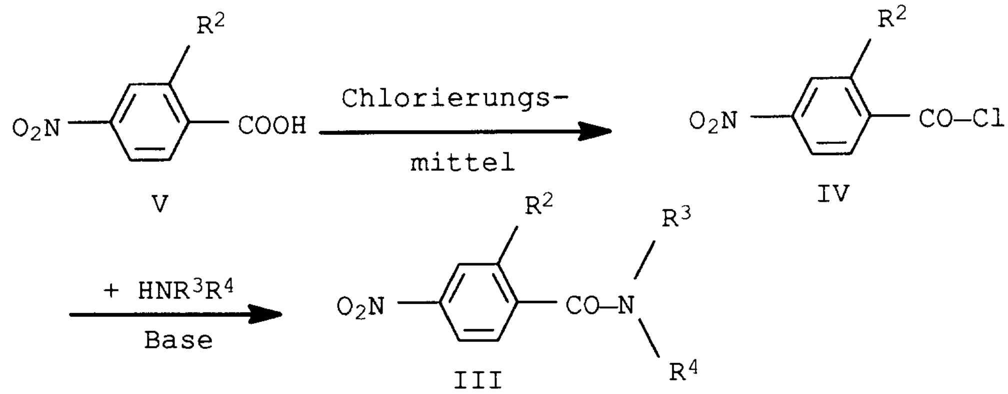


V IV III

Normalerweise führt man die Chlorierung in einem inerten Lösungsmittel durch, wobei aromatischen Kohlenwasserstoffe wie Toluol und Xylol, Ether wie Diethylether und Tetrahydrofuran und chlorierte Kohlenwasserstoffe wie Dichlormethan und Chloroform geeignet sind.

Besonders vorteilhaft führt man die Chlorierung in Gegenwart eines Katalysators durch, wofür z.B. Dimethylformamid oder Phosphinoxide geeignet sind.

Als Hilfsbasen für die Umsetzung von IV mit $HNR^3R^4$ eignen sich z.B. überschüssiges Amin $HNR^3R^4$, tertiäre Amine wie Triethylamin und Pyridin oder anorganische Basen, z.B. Alkalimetallcarbonate wie Natrium- und Kaliumcarbonat.

b) Umsetzung von Säurechloriden VI mit Aminen ($HNR^3R^4$):

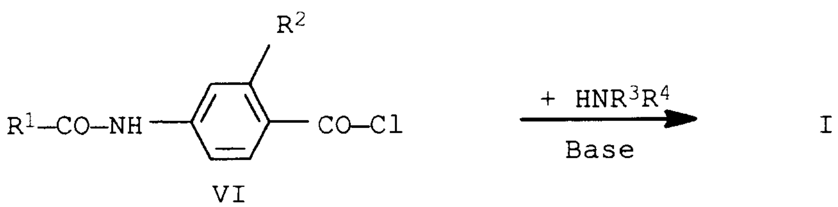


VI I

Die Umsetzung erfolgt normalerweise in einem inerten Lösungs- oder Verdünnungsmittel in Gegenwart einer Base, wobei als Base z.B. überschüssiges Amin $HNR^3R^4$, ein tertiäres Amins wie Triethylamin und Pyridin oder eine anorganische Base, z.B. Alkalimetallcarbonate wie Natrium- und Kaliumcarbonat, in Betracht kommen.

Vorteilhaft setzt man alle Komponenten (Säurechlorid VI, Amin $HNR^3R^4$ und tertiäres Amin/anorganische Base) in etwa stöchiometrischem Verhältnis ein, jedoch kann in manchen Fällen auch ein Überschuß der einen oder anderen Komponente, bis etwa 10 mol-%, empfehlenswert sein.

Verwendet man das Amin $HNR^3R^4$ als Base, so liegt diese Kompnente in einem größerem Überschuß vor.

Die Säurechloride der Formel VI sind ihrerseits durch Halogenierung von Carbonsäuren der Formel VII mit üblichen Halogenierungsreagenzien wie Phosgen oder Oxalylchlorid erhältlich:

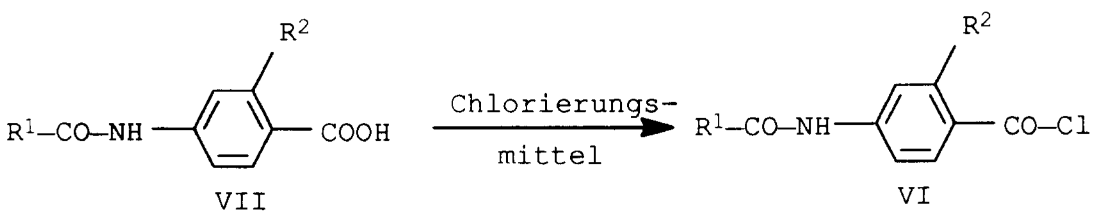


VII VI

Normalerweise führt man die Reaktion in einem inerten Lösungsmittel durch, wobei aromatischen Kohlenwasserstoffe wie Toluol, Ether wie Tetrahydrofuran und chlorierte Kohlenwasserstoffe wie Dichlormethan geeignet sind.

Besonders vorteilhaft führt man die Reaktion in Gegenwart eines Katalysators durch, wofür z.B. Dimethylformamid oder Phosphinoxide geeignet sind.

Hierbei ist im allgemeinen eine Katalysatormenge von 0,05 bis 2,0 mol-%, bezogen auf das Halogenierungsreagenz, ausreichend.

Die Carbonsäuren der Formel VII wiederum erhält man durch Umsetzung der entsprechenden 4-Aminobenzoesäuren VIII mit einem Säurechlorid der Formel $R^1$-CO-Cl in Gegenwart von mindestens zwei Äquivalenten einer organischen oder anorganischen Base:

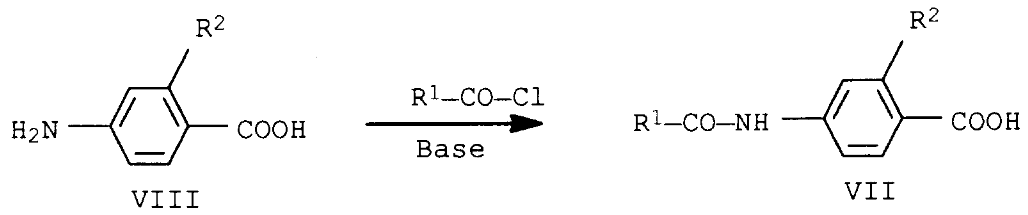


Als Basen eignen sich insbesondere organische Basen wie Triethylamin und Pyridin.

Als Lösungsmitteln kommen beispielsweise aromatischen Kohlenwasserstoffen wie Toluol und Xylol, halogenierten Kohlenwasserstoffen wie Dichlormethan und Chloroform sowie Ether wie Diethylether und Tetrahydrofuran in Betracht.

Die vorstehend beschriebenen Reaktionen werden alle zweckmäßigerweise bei Atmosphärendruck oder unter dem Eigendruck des jeweiligen Verdünnungsmittels vorgenommen.

Die Acylamino-benzamide der Formel I eignen sich als Fungizide.

Herstellungsbeispiel

Bicyclo[4.1.0]heptyl-1-carbonamido-3-chlor-4-N,N-dimethylbenzamid (Tabelle 2, Verb. Nr. 1)

Zu einer Lösung von 1,98 g (0,010 Mol) 2-Chlor-4-amino-N,N-dimethylbenzamid (J. Chem. Soc. 1960, 3163) und 1,10 g (0,011 Mol) Triethylamin in 30 ml Dichlormethan tropfte man bei 0 °C 1,74 g (0,011 Mol) Bicyclo[4.1.0]heptyl-1-carbonylchlorid {hergestellt aus der entsprechenden Säure [J. Org. Chem. 50 2128 (1985)] und Thionylchlorid} zu. Das Reaktionsgemisch wurde 1 Stunde bei 0 °C und dann 12 Stunden bei 20-25 °C gerührt, anschließend mit etwa 20 ml Wasser gewaschen und schließlich getrocknet. Nach Verdampfen des Lösungsmittels wurde das Rohprodukt (3,0 aus t.-Butylmethylether umkristallisiert. Ausbeute: 2,3 g (71,8 %); Fp.: 143-145 °C

In Tabelle 2 sind weitere Acylamino-benzamide I enthalten, die sich analog zu der Beispielsverbindung herstellen lassen:

Tabelle 2

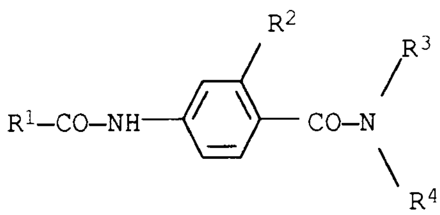

I

| Nr. | $R^1$ | $R^2$ | $R^3$ | $R^4$ | Fp. [°C] |
|---|---|---|---|---|---|
| I.01 | Bicyclo[4.1.0]hept-1-yl | Cl | $CH_3$ | $CH_3$ | 143-145 |
| I.02 | 2-Methyl-bicyclo[2.2.2]oct-2-yl | Cl | $CH_3$ | $CH_3$ | |
| I.03 | 2-Methyl-bicyclo[2.2.1]hept-2-yl | Cl | $CH_3$ | $CH_3$ | 185-187 |
| I.04 | 2-Methyl-2.3.5.6-tetrahydro-4H-pyran-2-yl | Cl | $CH_3$ | $CH_3$ | Öl |

Die Acylamino-benzamide I zeichnen sich durch eine hervorragende Wirksamkeit gegen ein breites Spektrum von pflanzenpathogenen Pilzen, insbesondere aus der Klasse der Phycomyceten, aus. Sie sind z.T. systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:

* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:

- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die fungiziden Mittel enthalten im allgemeinen zwischen 0,1 und 95, vorzugsweise zwischen 0,5 und 90 Gew.% Wirkstoff.

Beispiele für solche Zubereitungen sind:

I. eine Lösung aus 90 Gew.-Teilen der Verbindung Nr. I.01 und 10 Gew. -Teilen N-Methyl-α-pyrrolidon, die zur Anwendung in Form kleinster Tropfen geeignet ist;

II. eine Mischung aus 20 Gew.-Teilen der Verbindung Nr. I.02, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der L$sung in Wasser erhält man eine Dispersion.

III. eine wäßrige Dispersion aus 20 Gew.-Teilen der Verbindung Nr. I.03, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. I.04, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. I.01, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. I.03 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. I.03, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. I.04, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. I.01, 2 Gew.-Teilen des Calciumsalzes der Dodecylbenzolsulfonsäure, 8 Gew.-Teilen Fettalkohol-polyglykolether, 20 Gew.- Teilen des Natriumsalzes eines Phenolsulfonsäure-harnstoff-formaldehyd-Kondensates und 68 Gew.-Teilen eines paraffinischen Mineralöls.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie 2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon, 2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,

2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methylpyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiel

Wirksamkeit gegen Phytophthora infestans an Tomaten

Blätter von Topfpflanzen der Sorte "Große Fleischtomate" wurden mit wässriger Spritzbrühe, die 80 Gew.-% Wirkstoff und 20 Gew.-% Emulgiermittel in der Trockensubstanz enthielt, besprüht. Nach 24 Std. infizierte man die Blätter mit einer Zoosporenaufschwemmung des Pilzes Phytophthora infestans. Die Pflanzen wurden dann in einer wasserdampfgesättigten Kammer bei Temperaturen zwischen 16 und 18 °C aufgestellt. Nach 6 Tagen hatte sich die Krankheit auf den unbehandelten, jedoch infizierten Kontrollpflanzen so stark entwickelt, daß die fungizide Wirksamkeit der getesteten Verbindungen beurteilt werden konnte.

Gegenüber dem Kontrollversuch (keine Behandlung, 80 % Pilzbefall) zeigte sich, daß die mit den Wirkstoffen I.01 und I.04 behandelten Pflanzen nur einen Pilzbefall von 0-5 % hatten.

## Patentansprüche

**1.** Acylamino-benzamide der Formel I

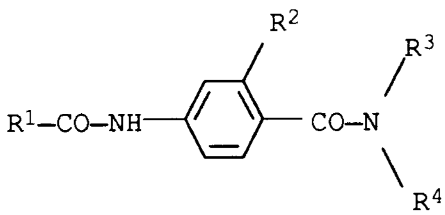

I

in der die Substituenten folgende Bedeutung haben:

- $R^1$ Bicyclo[4.1.0]hept-1-yl, Bicyclo[3.1.0]hex-1-yl, 2-($C_1$-$C_3$-Alkyl)-bicyclo[2.2.2]oct-2-yl, 2-($C_1$-$C_3$-Alkyl)-bicyclo[2.2.2]oct-5-en-2-yl, 2-($C_1$-$C_3$-Alkyl)-bicyclo[2.2.1]hept-2-yl, 2-($C_1$-$C_3$-Alkyl)-bicyclo[2.2.1]hept-5-en-2-yl, 6-($C_1$-$C_3$-Alkyl)-5,6-dihydro-4H-pyran-6-yl oder 2-($C_1$-$C_3$-Alkyl)-2.3.5.6-tetrahydro-4H-pyran-2-yl;
- $R^2$ Halogen, Cyano, Nitro, $C_1$-$C_4$-Alkyl, $C_2$-$C_4$-Alkenyl, $C_2$-$C_4$-Alkinyl, $C_1$-$C_4$-Halogenalkyl, $C_1$-$C_4$-Alkoxy, $C_1$-$C_4$-Alkoxycarbonyl, $C_1$-$C_4$-Alkylcarbonyl, Formyl oder Carboxy;
- $R^3$,$R^4$ unabhängig voneinander Cyano, $C_1$-$C_4$-Alkyl, $C_1$-$C_4$-Halogenalkyl, $C_3$-$C_6$-Alkenyl, $C_3$-$C_6$-Halogenalkenyl, $C_3$-$C_6$-Alkinyl, $C_3$-$C_6$-Halogenalkinyl, $C_1$-$C_4$-Alkoxy, $C_1$-$C_4$-Alkoxy-$C_1$-$C_4$-alkyl, $C_1$-$C_4$-Alkylthio-$C_1$-$C_4$-alkyl, $C_3$-$C_6$-Cycloalkyl oder $C_3$-$C_6$-Cycloalkyl-$C_1$-$C_4$-alkyl, wobei die Cycloalkylringe der letzten beiden Substituenten unsubstituiert sein oder ihrerseits ein bis drei $C_1$-$C_4$-Alkylreste tragen können, oder
- $R^3$ und $R^4$ zusammen mit dem Stickstoffatom, an das sie gebunden sind, einen Azetidin-, Pyrrolidin-, Piperidin- oder Morpholin-Ring, wobei die Ringe unsubstituiert sein oder ein bis zwei $C_1$-$C_4$-Alkylreste tragen können.

**2.** Verwendung der Acylamino-benzamide I gemäß Anspruch I als Fungizide.

**3.** Fungizides Mittel, enthaltend flüssige oder feste Trägerstoffe und eine fungizid wirksame Menge mindestens eines Acylamino-benzamids der Formel I gemäß Anspruch 1.

**4.** Verfahren zur Bekämpfung von Pilzen, dadurch gekennzeichnet, daß man eine fungizid wirksame Menge mindestens eines Acylamino-benzamids I gemäß Anspruch 1 auf Pilze, vom Pilzbefall bedrohte Pflanzen, deren Lebensraum oder auf das Saatgut der bedrohten Pflanzen einwirken läßt.

Europäisches Patentamt

# EUROPÄISCHER RECHERCHENBERICHT

Nummer der Anmeldung
EP 94 11 8847

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP-A-0 470 711 (IMPERIAL CHEMICAL INDUSTRIES PLC;UK ) 12. Februar 1992<br>* Seite 11 - Seite 13 *<br>--- | 1-4 | C07C237/42<br>C07D309/08<br>A01N37/22<br>A01N43/16 |
| D,A | EP-A-0 381 330 (IMPERIAL CHEMICAL INDUSTRIES PLC;UK ) 8. August 1990<br>* Ansprüche *<br>----- | 1-4 | |
| | | | **RECHERCHIERTE SACHGEBIETE (Int.Cl.6)** |
| | | | C07C<br>C07D |

Der vorliegende Recherchenbericht wurde für alle Patentansprüche erstellt

| Recherchenort | Abschlußdatum der Recherche | Prüfer |
|---|---|---|
| DEN HAAG | 6. März 1995 | Pauwels, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..........................................................................
& : Mitglied der gleichen Patentfamilie, übereinstimmendes Dokument

EPO FORM 1503 03.82 (P04C03)